# EUROPÄISCHE PATENTSCHRIFT

(11) **EP 3 012 014 B1**
(45) Veröffentlichungstag und Bekanntmachung des Hinweises auf die Patenterteilung: **31.05.2017**
(21) Anmeldenummer: 14189728.0
(22) Anmeldetag: 21.10.2014
(51) Int. Cl.: B01F 1/00, A61M 1/16, B01F 15/00, G01N 9/04, G01F 23/02

(54) **Flüssigkeitsbehandlungs-Anlage**
Liquid treatment system
Installation de traitement de liquide

(43) Veröffentlichungstag der Anmeldung: 27.04.2016
(73) Patentinhaber: Dumschat, Christoph, 26789 Leer (DE)
(72) Erfinder: Dumschat, Christoph, 26789 Leer (DE)
(74) Vertreter: Patentanwälte ter Smitten Eberlein-Van Hoof Rütten Partnerschaftsgesellschaft mbB

(56) Entgegenhaltungen:
- EP-A1- 1 154 244
- EP-A1- 2 623 188
- DE-A1-102004 026 477
- DE-B3- 10 313 965
- FR-A- 1 394 100
- US-A- 3 004 544

## Beschreibung

Die Erfindung bezieht sich auf eine Flüssigkeitsbehandlungs-Anlage zur dichteverändernden Behandlung einer Flüssigkeit mit einer Flüssigkeitsdichte-Bestimmungsvorrichtung.

Eine Flüssigkeitsbehandlungs-Anlage kann beispielsweise eine Anlage zur Herstellung eines Dialysekonzentrats sein, wie sie aus DE 103 13 965 B3 bekannt ist. In dieser Flüssigkeitsbehandlungs-Anlage wird aus einem Trockenkonzentrat und Wasser ein saures Dialyseflüssigkeits-Konzentrat erzeugt. Hierzu wird in einen Flüssigkeitsspeicher, einem sogenannten Vorlagebehälter, zunächst eine genau abgemessene Wassermenge eingefüllt. Das Wasser aus dem Flüssigkeitsspeicher wird anschließend mit dem Trockenkonzentrat vermischt, wobei das Trockenkonzentrat in dem Wasser gelöst wird, wodurch sich die Dichte der Flüssigkeit verändert. Für die exakte Ermittlung der in den Flüssigkeitsspeicher eingelaufenen Wassermenge ist im Wasserzulauf ein präziser Flusssensor angeordnet, mit dem in einer Anlagensteuerung die eingelaufene Wassermenge aufsummierend ermittelt wird. Um das exakte Mischungsverhältnis, also die Konzentration des Trockenkonzentrats und seiner Bestandteile in der gesamten Flüssigkeitsmenge bestimmen zu können, ist in einer Flüssigkeitsleitung der Anlage ein hochpräziser Dichtesensor angeordnet.

Sowohl der Flusssensor als auch der Dichtesensor stellt jeweils ein teures und sensibles Bauteil dar, wobei sowohl die Flüssigkeitsmenge als auch die Flüssigkeitsdichte mit diesem Sensoren nur indirekt gemessen wird, also grundsätzlich einer messprinzip-bedingten Unsicherheit unterliegt.

EP 1 154 244 A1 offenbart eine Anlage bzw. ein Verfahren zur Messung des Füllstandes einer Flüssigkeit in einem Behälter, wobei der Flüssigkeitspegel in einem Füllstandsmessrohr ermittelt wird. Bei vollständig gefülltem Füllstandsmessrohr kann auch die Dichte oder die Konzentration einer Flüssigkeit gemessen werden.

FR 1 394 100 offenbart ebenfalls eine Füllstandsmessung für einen Flüssiggas-Tank, wobei das Flüssiggas in einem Füllstandsrohr steht. Die Temperatur des Flüssiggases in dem Füllstandsrohr wird an einem Thermometer an dem Füllstandsrohr abgelesen, und der an dem Füllstandsrohr abgelesene Pegel wird entsprechend der Temperatur anhand einer Tafel korrigiert.

Aufgabe der Erfindung vor diesem Hintergrund ist es, eine Flüssigkeitsbehandlungs-Anlage mit einer verbesserten Flüssigkeitsdichte-Bestimmungsvorrichtung zu schaffen.

Diese Aufgabe wird erfindungsgemäß gelöst mit einer Flüssigkeitsbehandlungs-Anlage mit einer Flüssigkeitsdichte-Bestimmungsvorrichtung mit den Merkmalen des Anspruchs 1.

Die erfindungsgemäße Flüssigkeitsdichte-Bestimmungsvorrichtung weist ein vertikal bewegliches Flüssigkeits-Messgefäß mit einem bekannten Füllvolumen auf, das gegenüber dem gesamten Flüssigkeitsvolumen in der Anlage klein sein kann, also beispielsweise weniger als 10 % oder weniger als 5 % des gesamten Flüssigkeitsvolumens in der Anlage betragen kann. Das Flüssigkeits-Messgefäß weist einen Flüssigkeits-Einlass auf, durch den die Flüssigkeit in das Messgefäß einfließen kann. In den Flüssigkeits-Einlass mündet eine nicht-starre Füllleitung, die aufgrund ihrer Nicht-Starrheit widerstandsfrei die vertikale Beweglichkeit des Flüssigkeits-Messgefäßes ermöglicht. Die nicht-starre Füllleitung kann beispielsweise als filexibler Schlauch ausgebildet sein kann jedoch auch aus einzelnen starren Elementen bestehen, die beispielsweise teleskop- oder gelenkartig miteinander verbunden sind. Dem Messgefäß ist ein Füllsensor zugeordnet, der die vollständige Füllung des Messgefäßes mit der Flüssigkeit detektiert. Der Füllsensor gibt ein Füllsignal aus, wenn das Flüssigkeits-Messgefäß mit dem bekannten Füllvolumen an Flüssigkeit vollständig gefüllt ist. Ferner ist dem Messgefäß ein Temperatursensor zugeordnet, der die Flüssigkeitstemperatur der Flüssigkeit in dem Messgefäß detektiert. Die Flüssigkeits-Temperatur ist für die Dichteermittlung essenziell, da die Dichte einer Flüssigkeit von Ihrer Temperatur abhängt.

Ferner ist dem Flüssigkeits-Messgefäß eine elektronische Waage zugeordnet, die das Flüssigkeits-Messgefäß trägt und die die Flüssigkeitsmasse in dem Messgefäß ermittelt. Die Flüssigkeitsmasse ergibt sich hierbei aus der Differenz der zuvor ermittelten Leermasse des leeren Messgefäßes und der Gesamtmasse des vollständig mit der Flüssigkeit gefüllten Messgefäßes.

Eine Anlagensteuerung weist einen Dichteermittler auf, der die physikalische Dichte der Flüssigkeit aus dem bekannten Füllvolumen des Messgefäßes, der von dem Temperatursensor ermittelten Flüssigkeitstemperatur und der von der elektronischen Waage gemessenen Flüssigkeitsmasse ermittelt, sobald bzw. wenn der Füllsensor die vollständige Füllung des Messgefäßes mit der Flüssigkeit detektiert. Die Dichte wird mit dieser Vorrichtung bzw. mit diesem Verfahren also im physikalischen Sinne direkt ermittelt, nämlich aus der Flüssigkeitsmasse und dem bekannten Füllvolumen. Da die Temperatur der Flüssigkeit ebenfalls bekannt ist, kann die Flüssigkeitsdichte normiert werden, so dass aus der auf diese Weise normierten Flüssigkeitsdichte die exakte Konzentration eines Analyts in der Flüssigkeit ermittelt werden kann, beispielsweise die exakte Konzentration eines Dialyseflüssigkeit-Konzentrats bzw. seiner Substanzen in der Flüssigkeit ermittelt werden kann.

Mit der beschriebenen Vorrichtung bzw. dem beschriebenen Verfahren lässt sich auf technisch relativ einfache Weise eine sehr hohe Messgenauigkeit und Messsicherheit realisieren, wobei die Gesamtkosten der für die Dichteermittlung zusätzlich erforderlichen Bauteile relativ gering sind. Über die Sensorik hinaus, die beispielsweise in einer Flüssigkeitsbehandlungs-Anlage zur Erzeugung eines Dialysekonzentrats ohnehin vorhanden sind, ist vorliegend nur eine elektronische Waage erforderlich, die als Konfektionsartikel preiswert erhältlich ist.

Gemäß einer bevorzugten Ausgestaltung weist die Flüssigkeitsbehandlungs-Anlage einen Flüssigkeitsspeicher mit bekannten Volumenmaßen auf. Der Flüssigkeitsspeicher ist beispielsweise in vertikaler Richtung querschnittskonstant ausgebildet, so dass ein linearer Zusammenhang zwischen der Füllhöhe und dem Füllvolumen besteht. Fluidisch parallel zu dem Flüssigkeitsspeicher ist das als Steigrohr ausgebildete Flüssigkeits-Messgefäß vorgesehen, das fluidisch mit dem feststehenden Flüssigkeitsspeicher über eine nicht-starre Kommunikationsleitung kommuniziert. Die Füllhöhe in dem Steigrohr entspricht exakt der Füllhöhe in dem Flüssigkeitsspeicher. An dem Steigrohr kann also der Füllstand des Flüssigkeitsspeichers "abgelesen" werden. Die nicht-starre Kommunikationsleitung kann identisch mit der nicht-starren Füllleitung sein, kann jedoch auch eine von der Füllleitung separat ausgebildete Leitung sein.

Die Anlagensteuerung weist bevorzugt einen Volumenermittler auf, der aus den bekannten Flüssigkeitsspeicher-Volumenmaßen, der physikalischen Dichte der Flüssigkeit und der gemessenen Flüssigkeitsmasse das Flüssigkeitsvolumen der Flüssigkeit in dem Flüssigkeitsspeicher ermittelt, wenn der Flüssigkeitspegel in dem Steigrohr dem Flüssigkeitspegel in dem Flüssigkeitsspeicher entspricht.

Die für die Dichteermittlung erforderliche elektronische Waage kann also zusätzlich auch für eine exakte Volumenermittlung des Flüssigkeitsvolumens in dem Flüssigkeitsspeicher genutzt werden. Unter Einbeziehung der Flüssigkeitstemperatur kann das Flüssigkeitsvolumen normiert werden.

Vorzugsweise weist die Flüssigkeitsbehandlungs-Anlage eine Füllpumpe auf, die stromabwärts der Füllleitung angeordnet ist. Die Füllpumpe ist insbesondere dazu erforderlich, das Flüssigkeits-Messgefäß vollständig mit der Flüssigkeit füllen zu können, selbst wenn der statische Druck der Flüssigkeit in der Anlage hierzu nicht ausreichen würde. Insbesondere bei Ausbildung des Flüssigkeit-Messgefäßes als Steigrohr ist eine Füllpumpe erforderlich, um das Steigrohr in voller Höhe zu füllen.

Vorzugsweise ist an dem unteren Ende des Messgefäßes ein Leersensor angeordnet, der die vollständige Entleerung des Messgefäßes detektiert. Immer dann, wenn das Messgefäß vollständig entleert ist, was durch den Leersensor gemeldet wird, kann mit der elektronischen Waage eine Leermessung durchgeführt werden, um das exakte Leergewicht des Flüssigkeits-Messgefäßes und aller von der elektronischen Waage getragenen Bestandteile zu ermitteln. Diese Leermessung kann regelmäßig durchgeführt werden, um gravierende Fehler bei der Dichteermittlung und/oder der Volumenermittlung zu vermeiden.

Vorzugsweise ist am oberen Ende des Messgefäßes eine nicht-starre Überlaufleitung vorgesehen, die oben in den Flüssigkeitsspeicher mündet. Die Füllleitung mündet vorzugsweise am unteren Ende des Messgefäßes in das Messgefäß. Auf diese Weise kann das Messgefäß kontinuierlich von der Flüssigkeit von unten nach oben durchströmt werden, so dass Dichtemessungen zeitlich relativ engmaschig durchgeführt werden können.

Gemäß einer bevorzugten Ausgestaltung weist die Flüssigkeitsbehandlungs-Anlage stromaufwärts der Füllpumpe eine Leitungs-Abzweigung auf und weist der Flüssigkeitsspeicher mindestens einen Speicherzulauf auf, wobei die Leitungs-Abzweigung und der Speicherzulauf jeweils eine fluidische Verbindung zu einem Mischbehälter dazwischen herstellen, in dem ein Dialysat-Trockenkonzentrat gelagert ist, das in der Anlage in dem Wasser bzw. der Flüssigkeit gelöst wird. Die Flüssigkeitsbehandlungs-Anlage ist also bevorzugt eine Anlage zur Herstellung eines Dialyseflüssigkeits-Konzentrats, wie sie beispielsweise aus der DE 103 13 965 B3 bekannt ist. Hierbei kann die Füllpumpe auch die übrigen Pumparbeiten in der Anlage übernehmen, so dass keine weitere Pumpe erforderlich ist.

Im Folgenden wird unter Bezugnahme auf die Zeichnungen ein Ausführungsbeispiel der Erfindung näher erläutert.

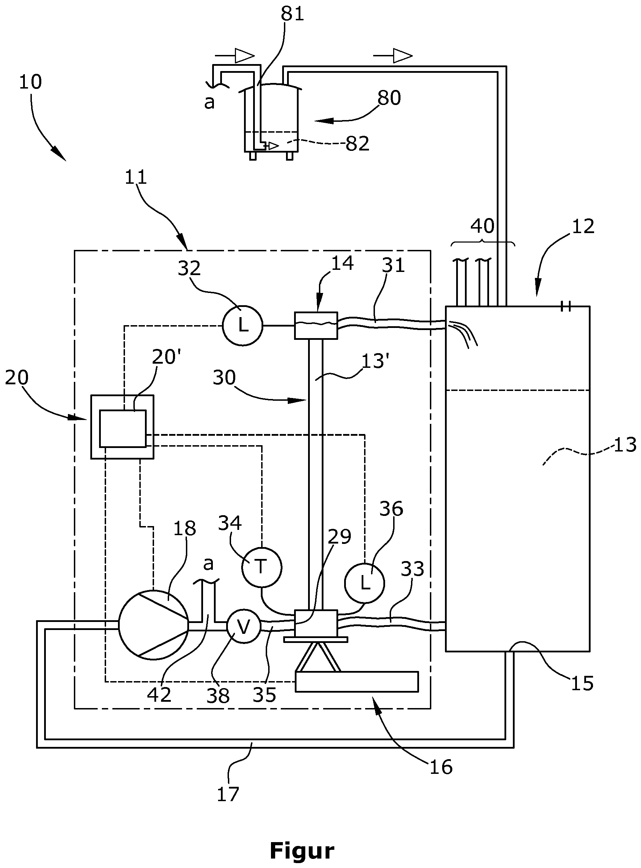

Die Zeichnung zeigt eine Flüssigkeitsbehandlungs-Anlage zur Dichte verändernden Behandlung einer Flüssigkeit mit einer flüssigkeitsdichte-Bestimmungsvorrichtung.

Die Flüssigkeitsbehandlungs-Anlage 10 ist vorliegend eine Anlage zur Herstellung eines Dialyseflüssigkeits-Konzentrats, wie sie aus der DE 103 13 965 B3 bekannt ist. Die Flüssigkeitsbehandlungs-Anlage 10 weist unter anderem einen als sogenannter Vorlagebehälter ausgebildeten Flüssigkeitsspeicher 12, einen Wechselbehälter 80 mit einem Dialyseflüssigkeits-Trockenkonzentrat 82 und eine Flüssigkeitsdichte-Bestimmungsvorrichtung 11 auf. Über einen Speicherzulauf 40 des Flüssigkeitsspeichers 12 wird zunächst Wasser in einer Sollmenge bzw. in einem Sollvolumen in den Flüssigkeitsspeicher 12 eingefüllt. Anschließend wird das Wasser zu dem Wechselbehälter 80 gepumpt, wodurch das Trockenkonzentrat 82 in dem Wasser gelöst wird. Das Wasser bzw. die auf diese Weise entstehende Flüssigkeit wird schließlich so lange zwischen dem Flüssigkeitsspeicher 12 und dem Wechselbehälter 80 im Kreis gepumpt, bis das Trockenkonzentrat homogen in dem gesamten Flüssigkeitsvolumen gelöst ist. Sobald das Trockenkonzentrat homogen in dem gesamten Flüssigkeitsvolumen gelöst ist, wird das gesamte Flüssigkeitsvolumen in den Flüssigkeitsspeicher 12 gepumpt. Das auf diese Weise entstandene Flüssigkeitsvolumen ist ein Dialyseflüssigkeits-Konzentrat, das in einem Dialysegerät erneut mit Wasser zu einer Dialyseflüssigkeit gemischt bzw. verdünnt wird.

Für den gesamten vorbeschriebenen Herstellungsprozess ist zur Überwachung die exakte Bestimmung der Flüssigkeitsdichte sowie des Flüssigkeitsvolumens erforderlich. Hierzu dient die Flüssigkeitsdichte-Bestimmungsvorrichtung 11, die im Wesentlichen ein als Steigrohr 30 ausgebildetes Messgefäß 14, eine Füllpumpe 18, eine elektronische Waage 16 und einen Dichteermittler 20' in einer Anlagensteuerung 20 aufweist.

Das Messgefäß 14 ist ein vertikal stehendes und zylindrisches Steigrohr 30, das an seinem oberen Ende, dem Kopf, einen Füllsensor 32 und an seinem unteren Ende, dem Fuß, einen Leersensor 36, sowie einen Temperatursensor 34 aufweist. Der Leersensor 36 und der Füllsensor 32 können jeweils als optische Sensoren ausgestaltet sein, die die Anwesenheit bzw. Abwesenheit von Flüssigkeit 13' am Messort feststellen. Andere Sensortypen, z.B. Schwimmerschalter sind jedoch auch geeignet.

An dem Fußende des Steigrohrs 30 ist dieses über eine flexible Kommunikationsleitung 33 mit dem unteren Bereich des Flüssigkeitsspeichers 12 fluidisch verbunden. Ferner ist das Steigrohr 30 an seinem Fußende über eine separate Füllleitung 35, die ebenfalls flexibel ausgebildet ist, mit einer Füllpumpe 18 fluidisch verbunden. Zwischen der Füllpumpe 18 und dem Steigrohr-Fußende ist ein Sperrventil 38 angeordnet. Ferner ist zwischen dem Sperrventil 38 und der Füllpumpe 18 eine fluidische Abzweigung 42 angeordnet, die in den Flüssigkeitseinlauf 81 des Wechselbehälters 80 mündet. An dem Kopfende des Steigrohrs 30 ist eine flexible Überlaufleitung 31 vorgesehen, die in den oberen Bereich des Flüssigkeitsspeichers 12 mündet. Das Messgefäß 1,4 bzw. das Steigrohr 30 ist vertikal im erforderlichen Maße beweglich und steht auf der Waage 16 bzw. hängt an der Waage 16, die das gesamte Gewicht des Messgefäßes 14 bzw. des Steigrohrs 30 trägt. Die flexiblen Leitungen 31,33,35 sind derart flexibel, dass sie die vertikale Beweglichkeit des Steigrohrs 30 allenfalls vernachlässigbar beeinflussen, also keine nennenswerten vertikalen Kräfte auf das Steigrohr 30 ausüben.

Das Flüssigkeit-Messgefäß 14 bzw. das Steigrohr 30 hat ein exakt bekanntes Füllvolumen F von beispielsweise 3,0 I. Da das Füllvolumen 11 exakt bekannt ist, kann mit Hilfe der elektronischen Waage 16 eine exakte Dichtebestimmung der physikalischen Dichte D der Flüssigkeit 13' in dem Steigrohr 30 vorgenommen werden, wenn das Messgefäß 14 bzw. das Steigrohr 30 vollständig gefüllt ist. Für eine Dichtebestimmung wird die Füllpumpe 18 bei geöffneten Sperrventil 38 eingeschaltet, so dass aus dem Flüssigkeitsspeicher-Auslass 15 durch die Füllpumpe 18 Flüssigkeit in einen fußseitigen Einlass 29 des Messgefäßes 14 gepumpt wird. Die Kommunikationsleitung 33 wird entweder durch ein weiteres, nicht dargestelltes, Sperrventil geschlossen, oder aber ist von so geringem Strömungsquerschnitt, dass durch die Kommunikationsleitung 33 nur ein vergleichsweise geringer Flüssigkeitsfluss in Richtung Flüssigkeitsspeicher 12 auftritt. Auf diese Weise wird das Messgefäß 14 vollständig gefüllt, wobei der Überschuss über die Überlaufleitung 31 zurück in den Flüssigkeitsspeicher 12 läuft. Sobald das Messgefäß 14 vollständig mit Flüssigkeit 13' gefüllt ist, wird dies von dem Füllsensor 32 detektiert und an den Dichteermittler 20' gemeldet. In diesem Moment ermittelt die Waage 16 die Flüssigkeitsmasse m aus der Differenz des bekannten Leergewichts des Messgefäßes 14 und des gemessenen Gesamtgewichts des gefüllten Messgefäßes 14. Ferner erfasst der Dichteermittler 20' die aktuelle Flüssigkeitstemperatur t, die von dem Temperatursensor 34 ermittelt und gemeldet wird.

Der Dichteermittler 20' ermittelt schließlich aus der von der Waage gemeldeten Flüssigkeitsmasse m und dem bekannten Füllvolumen F die Dichte D Flüssigkeit 13' in dem Messgefäß 14. Unter Einbeziehung der Flüssigkeitstemperatur t wird die errechnete Flüssigkeitsdichte normiert, beispielsweise auf eine Flüssigkeitstemperatur von 20 °C bezogen. Aus der normierten physikalischen Dichte D lässt sich dann unmittelbar und exakt auf die Konzentration K des Trockenkonzentrats bzw. seiner Bestandteile in der Flüssigkeit schließen. Ferner lässt sich durch die Durchführung mehrerer Dichteermittlungen auch eine genaue Aussage über die Dichte-Homogenität der Flüssigkeit 13,13' machen. Sobald die Homogenität eine vorbestimmte Qualität erreicht, wird der Mischprozess beendet, und steht das Dialyseflüssigkeits-Konzentrat für die Therapie zur Verfügung.

Die Flüssigkeitsdichte-Bestimmungsvorrichtung 11 wird auch zur Bestimmung des Flüssigkeitsvolumens bzw. der Flüssigkeitsmasse der Flüssigkeit 13 in dem Flüssigkeitsspeicher 12 benutzt. Hierzu wird das Sperrventil 38 geschlossen und/oder die Füllpumpe 18 abgeschaltet. In dem Steigrohr 30, das horizontal ungefähr mit dem Flüssigkeitsspeicher 12 fluchtet, hat die darin befindliche Flüssigkeit 13' exakt den gleichen Pegel die die Flüssigkeit 13 in dem Flüssigkeitsspeicher 12. Die Masse der betreffenden Flüssigkeitssäule der Flüssigkeit 13' in dem Steigrohr 30 wird über die elektronische Waage 16 ermittelt und einem Volumenermittler 20' gemeldet, der vorliegend technisch identisch ist mit dem Dichteermittler 20', grundsätzlich aber auch separat von dem Dichteermittler 20' ausgebildet sein kann. Aus der von der Waage gemessenen Flüssigkeitsmasse m, dem bekannten Steigrohr-Füllvolumen F, der durch den Temperatursensor 34 ermittelten Flüssigkeitstemperatur t, der physikalischen Dichte D der betreffenden Flüssigkeit 13' für die gemessene Temperatur und den bekannten Flüssigkeitsspeicher-Volumenmaßen wird das exakte Flüssigkeitsvolumen der Flüssigkeit 13 in dem Flüssigkeitsspeicher 12 errechnet.

Die Flüssigkeitsspeicher-Volumenmaße geben das Querschnitts-Profil über die Höhe an, das in der Regel konstant ist, da der Flüssigkeitsspeicher 12 vorzugsweise einen über die Höhe konstanten, beispielsweise zylindrischen, Querschnitt aufweist.

Mit der vorbeschriebenen Volumenbestimmung kann sowohl die zu Prozessbeginn in den Flüssigkeitsspeicher 12 eingefüllte Wassermasse als auch die am Prozessende in dem Flüssigkeitsspeicher 12 gespeicherte Flüssigkeitsmasse an Dialyseflüssigkeits-Konzentrat bestimmt werden, da für beide Flüssigkeiten die exakte Dichte bekannt ist bzw. mit der beschriebenen Dichtebestimmung zuvor bestimmt werden kann.

## Patentansprüche

1. Flüssigkeitsbehandlungs-Anlage (10) zur dichteverändernden Behandlung einer Flüssigkeit (13), mit einer Flüssigkeitsdichte-Bestimmungsvorrichtung (11) mit:
einem vertikal beweglichen Flüssigkeits-Messgefäß (14) mit bekannten Füllvolumen (F) und mit einem Flüssigkeits-Einlass (29),
einer nicht-starren Füllleitung (35), die in den Flüssigkeits-Einlass (29) mündet,
einem Füllsensor (32), der die vollständige Füllung des Messgefäßes (14) mit der Flüssigkeit (13') detektiert,
einem Temperatursensor (34), der die Flüssigkeitstemperatur (t) der Flüssigkeit (13') in dem Messgefäß (14) detektiert,
einer elektronischen Waage (16), die das Flüssigkeits-Messgefäß (14) trägt und die Flüssigkeitsmasse (m) bestimmt, und
einer Anlagensteuerung (20) mit einem Dichteermittler (20'), der die physikalische Dichte (D) der Flüssigkeit (13') aus dem bekannten Füllvolumen (F), der gemessenen Flüssigkeitstemperatur (t), und der gemessenen Flüssigkeitsmasse (m) ermittelt, wenn der Füllsensor (32) die vollständige Füllung des Messgefäßes (14) mit der Flüssigkeit (13') detektiert.

2. Flüssigkeitsbehandlungs-Anlage (10) nach Anspruch 1, mit einem Flüssigkeitsspeicher (12) mit bekannten Volumenmaßen, wobei das Flüssigkeits-Messgefäß (14) als Steigrohr (30) ausgebildet ist und fluidisch mit dem Flüssigkeitsspeicher (12) über eine nicht-starre Kommunikationsleitung (33) kommuniziert, wobei die Anlagensteuerung (20) einen Volumenermittler (20') aufweist, der aus den bekannten Flüssigkeitsspeicher-Volumenmaßen, der physikalischen Dichte (D) der Flüssigkeit (13'), und der gemessenen Flüssigkeitsmasse (m) das Flüssigkeitsvolumen (V) der Flüssigkeit (13) in dem Flüssigkeitsspeicher (12) ermittelt, wenn der Flüssigkeitspegel in dem Steigrohr (30) dem Flüssigkeitspegel in dem Flüssigkeitsspeicher (12) entspricht.

3. Flüssigkeitsbehandlungs-Anlage (10) nach einem der vorangegangenen Ansprüche, wobei eine Füllpumpe (18) vorgesehen ist, die stromabwärts der Füllleitung (35) angeordnet ist.

4. Flüssigkeitsbehandlungs-Anlage (10) nach einem der vorangegangenen Ansprüche, wobei am unteren Ende des Messgefäßes (14) ein Leersensor (36) angeordnet ist, der die vollständige Entleerung des Messgefäßes (14) detektiert.

5. Flüssigkeitsbehandlungs-Anlage (10) nach einem der vorangegangenen Ansprüche, wobei am oberen Ende des Messgefäßes (14) eine nicht-starre Überlaufleitung (31) vorgesehen ist, die oben in den Flüssigkeitsspeicher (12) mündet.

6. Flüssigkeitsbehandlungs-Anlage (10) nach Anspruch 3, wobei stromaufwärts der Füllpumpe (18) eine Leitungs-Abzweigung (42) vorgesehen ist und der Flüssigkeitsspeicher (12) mindestens einen Speicherzulauf (40) aufweist, wobei die Leitungs-Abzweigung (42) und der Speicherzulauf (40) die fluidische Verbindung zu einem Mischbehälter (60) herstellen, in dem Dialysat-Trockenkonzentrat (62) in Wasser gelöst wird.

## Claims

1. Liquid treatment system (10) for a density changing treatment of a liquid (13), having a liquid density determination device (11) comprising:
a vertically movable liquid measuring container (14) with a known filling volume (F) and a liquid inlet (29),
a non-rigid filling line (35) opening into the filling inlet (29),
a filling sensor (32) sensing the complete filling of the measuring container (14) with the liquid (13'),
a temperature sensor (34) sensing the liquid temperature (t) of the liquid (13') in the measuring container (14),
electronic scales (16) carrying the liquid measuring container (14) and determining the liquid mass (m), and
a system control (20) comprising a density determination means (20') determining the physical density (D) of the liquid (13') from the known filling volume (F), the measured liquid temperature (t) and the measured liquid mass (m), when the filling sensor (32) senses the complete filling of the measuring container (14) with the liquid (13').

2. Liquid treatment system (10) of claim 1, having a liquid reservoir (12) with known volume measures, wherein the liquid measuring container (14) is formed as a riser (30) and is in fluid communication with the liquid reservoir (12) via a non-rigid communication line (33), wherein the system control (20) has a volume determination means (20') determining the liquid volume (V) of the liquid (13) in the liquid reservoir (12) from the known liquid reservoir volume measures, the physical density (D) of the liquid (13') and the measured liquid mass (m), when the liquid level in the riser (30) corresponds to the liquid level in the liquid reservoir (12).

3. Liquid treatment system (10) of one of the preceding claims, wherein a filling pump (18) is provided that is arranged downstream of the filling line (35).

4. Liquid treatment system (10) of one of the preceding claims, wherein an empty-level sensor (36) is arranged at the lower end of the measuring container (14), said sensor detecting the complete draining of the measuring container (14).

5. Liquid treatment system (10) of one of the preceding claims, wherein a non-rigid overflow line (31) is provided at the upper end of the measuring container (14), said line opening into the liquid reservoir (12) at the top.

6. Liquid treatment system (10) of claim 3, wherein a branch line (42) is provided upstream of the filling pump (18) and the liquid reservoir (12) has at least one reservoir inflow (40), wherein the branch line (42) and the reservoir inflow (40) establish fluidic communication with a mixing container (60) in which dry dialysate concentrate (62) is dissolved in water.

## Revendications

1. Installation de traitement de liquide (10) pour le traitement de changement de densité d'un liquide (13), avec un dispositif de détermination (11) de la densité d'un liquide (11) comportant:
un récipient de mesure de liquide (14), déplaçable dans le sens vertical, ayant un volume de remplissage (F) connu et une entrée de liquide (29),
un conduit de remplissage (35) non-rigide débouchant dans l'entrée de liquide (29),
un capteur de remplissage (32) détectant le remplissage complet du récipient de mesure de liquide (14) avec le liquide (13'),
un capteur de température (34) détectant la température (t) du liquide (13¹) dans le récipient de mesure de liquide (14),
une balance électronique (16) portant ledit récipient de mesure de liquide (14) et déterminant la masse du liquide (m), et
une commande de l'installation (20) avec un détermineur de densité (20') déterminant la densité physique (D) du liquide (13') à partir du volume de remplissage (F) connu, la température du liquide (t) mesurée et la masse du liquide (m) mesurée, quand le capteur de remplissage (32) détecte le remplissage complet du récipient de mesure de liquide (14) avec le liquide (13').

2. Installation de traitement de liquide (10) selon la revendication 1, avec un réservoir de liquide (12) ayant des mesures de volume connues, ledit récipient de mesure de liquide (14) étant en forme d'un tube vertical (30) et étant en communication fluidique avec le réservoir de liquide (12) par un conduit de communication (33) non-rigide, ladite commande de l'installation (20) comporte un détermineur de volume (20') déterminant le volume (V) du liquide (13) dans le réservoir de liquide (12) à partir des mesures de volume connues du réservoir de liquide, la densité physique (D) du liquide (13') et la masse du liquide (m) mesurée, quand le niveau de liquide dans le tube vertical (30) correspond au niveau de liquide dans le réservoir de liquide (12).

3. Installation de traitement de liquide (10) selon l'une quelconque des revendications précédentes, dans laquelle une pompe de remplissage (18) est prévue, qui est disposée en aval du conduit de remplissage (35).

4. Installation de traitement de liquide (10) selon l'une quelconque des revendications précédentes, dans laquelle un capteur de vide (36) est disposé à l'extrémité basse du récipient de mesure de liquide (14), le capteur détectant la vidange complète du récipient de mesure de liquide (14).

5. Installation de traitement de liquide (10) selon l'une quelconque des revendications précédentes, dans laquelle un tuyau de trop-plein (31) non-rigide est prévu à l'extrémité haute du récipient de mesure de liquide (14), le tuyau débouchant en haut dans le réservoir de liquide (12).

6. Installation de traitement de liquide (10) selon la revendication 3, dans laquelle un branchement de conduit (42) est prévu en amont de la pompe de remplissage (18) et le réservoir de liquide (12) comporte au moins une arrivée du réservoir (40), le branchement de conduit (42) et l'arrivée du réservoir (40) réalisant la communication fluidique avec un récipient mélangeur (60) dans lequel de concentré sec de dialysat (62) est dissolu dans de l'eau.
